# FASCICULE DE BREVET EUROPEEN

(11) **EP 1 589 937 B1**
(45) Date de publication et mention de la délivrance du brevet: **19.09.2007**
(21) Numéro de dépôt: 04705098.4
(22) Date de dépôt: 26.01.2004
(51) Int. Cl.: A61K 8/19, A61Q 5/02

(54) **UTILISATION D UN PHOTOCATALYSEUR POUR LE TRAITEMENT DES CHEVEUX GRAS**
ANWENDUNG VON EINEM PHOTOKATALYSATOR ZUR BEHANDLUNG VON FETTENDEM HAAR
USE OF A PHOTOCATALYST FOR THE TREATMENT OF OILY HAIR

(30) Priorité: 27.01.2003 FR 0350005; 11.02.2003 US 445849 P
(43) Date de publication de la demande: 02.11.2005
(73) Titulaire: L'ORÉAL, 75008 Paris (FR)
(72) Inventeur: FACK, Géraldine, F-92300 Levallois (FR); SAMAIN, Henri, F-91570 Bievres (FR)
(74) Mandataire: Le Blainvaux Bellegarde, Françoise
(86) Numéro de dépôt international: PCT/FR2004/000172
(87) Numéro de publication internationale: WO 2004/075680

(56) Documents cités:
- EP-A- 0 988 853
- EP-A- 1 064 918
- WO-A-00/78281
- WO-A-03/078327
- FR-A- 2 806 907
- DATABASE WPI Section Ch, Week 199912 Derwent Publications Ltd., London, GB; Class D21, AN 1999-136693 XP002258048 & JP 11 005729 A (HYODO S) 12 janvier 1999 (1999-01-12)
- DATABASE CAPLUS [Online] CHEMICAL ABSTRACTS SERVICE, COLUMBUS, OHIO, US; 2003, XP002258040 extrait de STN Database accession no. 2003:582383 & JP 2003 212754 A (NATIONAL INSTITUTE OF ADVANCED INDUSTRIAL SCIENCE AND TECHNOLOGY) 30 juillet 2003 (2003-07-30)
- DATABASE CAPLUS [Online] CHEMICAL ABSTRACTS SERVICE, COLUMBUS, OHIO, US; 2000, XP002258041 extrait de STN Database accession no. 2000:316924 & JP 2000 136112 A (JAPAN) 16 mai 2000 (2000-05-16)
- DATABASE CAPLUS [Online] CHEMICAL ABSTRACTS SERVICE, COLUMBUS, OHIO, US; 2000, XP002258042 extrait de STN Database accession no. 2000:526690 & JP 2000 212012 A (EXCEL LIGHT Y.K.) 2 août 2000 (2000-08-02)
- DATABASE CAPLUS [Online] CHEMICAL ABSTRACTS SERVICE, COLUMBUS, OHIO, US; 2003, XP002258043 extrait de STN Database accession no. 2003:282686 & WO 03/029394 A (NATIONAL INSTITUTE OF ADVANCED INDUSTRIAL SCIENCE AND TECHNOLOGY) 10 avril 2003 (2003-04-10)
- DATABASE CAPLUS [Online] CHEMICAL ABSTRACTS SERVICE, COLUMBUS, OHIO, US; 2002, XP002258044 extrait de STN Database accession no. 2002:531125 & JP 2002 200148 A (TOA GOSEI CHEMICAL INDUSTRY CO) 16 juillet 2002 (2002-07-16)
- DATABASE CAPLUS [Online] CHEMICAL ABSTRACTS SERVICE, COLUMBUS, OHIO, US; 2003, XP002258045 extrait de STN Database accession no. 2003:257777 & JP 2003 095907 A (LION CORP.) 3 avril 2003 (2003-04-03)
- DATABASE CAPLUS [Online] CHEMICAL ABSTRACTS SERVICE, COLUMBUS, OHIO, US; 1998, XP002258046 extrait de STN Database accession no. 1998:197457 & WO 98/12048 A (HITACHI) 26 mars 1998 (1998-03-26)
- DATABASE CAPLUS [Online] CHEMICAL ABSTRACTS SERVICE, COLUMBUS, OHIO, US; 1998, XP002258047 extrait de STN Database accession no. 1998:555560 & JP 10 225393 A (TOTO) 25 août 1998 (1998-08-25)
- PATENT ABSTRACTS OF JAPAN vol. 2000, no. 09, 13 octobre 2000 (2000-10-13) & JP 2000 178595 A (SUMITOMO OSAKA CEMENT CO LTD), 27 juin 2000 (2000-06-27)

## Description

La présente invention a pour objet l'utilisation d'un photocatalyseur dans des compositions capillaires destinées à diminuer et/ou à éliminer les composés organiques indésirables sur les cheveux, notamment le sébum. L'invention a en particulier pour objet l'utilisation d'un photocatalyseur dans des compositions capillaires destinées à diminuer et/ou retarder le regraissage des cheveux et un procédé utilisant ce composé.

Les chevelures ont tendance à perdre certaines de leurs qualités sous l'action de facteurs tels que le regraissage naturel, la sueur, l'élimination de squames, la pollution, l'humidité et autres. Ces facteurs nuisent à l'aspect visuel et au toucher des cheveux. Ainsi, le regraissage (et éventuellement la pollution) alourdit les cheveux, lesquels ont tendance à se mettre en paquets. Les cheveux sont alors difficiles à coiffer, ils ont une brillance grasse désagréable et ont un toucher ciré également désagréable.

L'ampleur des conséquences de ces facteurs, presque tous inévitables, est très variable. Elle dépend par exemple de la qualité des cheveux, de leurs longueurs et de la coiffure adoptée.

Quoiqu'il en soit, pour lutter contre ces désagréments, on utilise des shampooings. En effet, le lavage avec des compositions détergentes est très efficace, il permet d'éliminer les salissures, les pellicules, de détendre les cheveux. Il est alors possible, au séchage, de replacer la chevelure dans la forme désirée. Cependant, l'effet bénéfique du shampooing s'estompe et on retrouve en quelques jours les problèmes décrits plus haut. En conséquence, on a tendance à augmenter la fréquence des shampooings.

Pour effectuer un shampooing, il faut avoir une source d'eau, de préférence chaude ou tiède.

Les compositions de shampooing sont à base de quantités importantes de tensioactifs qui peuvent générer des désagréments tels que des picotements au niveau du cuir chevelu ou des yeux.

Pour nettoyer plus rapidement les cheveux et éviter de mouiller les cheveux, on a déjà proposer d'utiliser des shampooings dits "secs". Cette technique consiste à pulvériser des particules absorbantes sur les cheveux puis à brosser activement la chevelure pour éliminer les particules. Mais en général, l'élimination complète des particules est très difficiles à obtenir. Les résultats ne sont pas très satisfaisants : la brillance des cheveux est faible et leur toucher est rêche.

Par ailleurs, il est connu qu'il est possible d'éliminer tout ou partie des corps étrangers ou indésirables du cheveu (dont sébum, pollution...) par oxydation.

La technique consiste à mettre un oxydant en présence des composés à éliminer sur le cheveu. Cette technique n'est pas satisfaisante car son effet est immédiat et ne peut se prolonger dans le temps compte tenu de l'absence de rémanence de l'oxydant liée à sa consommation totale lors de la réaction. Par ailleurs, les oxydants utilisés ont souvent une action néfaste sur les cheveux.

La présente invention a notamment pour but de résoudre les problèmes ci-dessus.

Plus précisément, la présente invention vise à proposer un procédé de traitement, notamment cosmétiques des cheveux humains pour diminuer et/ou retarder le regraissage des cheveux.

Or, à la suite d'importantes recherches menées sur la question, il a maintenant été trouvé par la Demanderesse, et ceci de façon tout à fait inattendue et surprenante, que ce but, et d'autres, pouvaient être atteints en mettant en oeuvre des compositions cosmétiques, destinée au traitement des cheveux comprenant dans un milieu cosmétiquement acceptable en particulier aqueux ou hydroalcoolique, au moins un photocatalyseur particulier. Cette découverte est à la base de la présente invention.

Les cheveux traités selon l'invention possèdent les caractéristiques des cheveux propres : le toucher est neutre (non gainé), les cheveux sont brillants (l'aspect gras a disparu), la chevelure est aérée et il n'y a pas de pellicules. Le regraissage est retardé, la chevelure garde son aspect propre pendant plusieurs jours, ce qui permet d'espacer les shampooings.

Elles présentent néanmoins pour avantage de conserver aux cheveux un toucher naturel et agréable.

La présente invention a pour objet l'utilisation d'un photocatalyseur dans des compositions capillaires destinées à diminuer et/ou à éliminer les composés organiques indésirables sur les cheveux humains, notamment le sébum, ledit photocatalyseur étant choisi parmi :
1) les composés insolubles dans l'eau choisis parmi:
   - V₂O₅, CeO₂, Nb₂O₅
   - WO₃, Na₄W₁₀O₃₂
   - MoO₃, MoS₂, RuO₂, Mn₂O₃, Bi₂O₃,
   - Fe₂O₃, Co₃O₄
   - CuInS₂, CuIn₅S₈,
   - les métaux tels que Ag, Cu, Au, Ni
2) les sulfures, les carbures et les phosphures insolubles dans l'eau choisis parmi:
   GaP, SiC, CdS et TiS
3) les composites insolubles dans l'eau choisis parmi les composites suivants : TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂ enrobé en surface de composés comprenant au moins un éléments choisi parmi Al, Zn, Zr, Cr, V, Nv, Fe, Cu, Co, Ni, Mn.

L'invention a en particulier pour objet l'utilisation d'un photocatalyseur dans des compositions capillaires destinées à diminuer et/ou retarder le regraissage des cheveux humains, ledit photocatalyseur étant choisi parmi :
1) les composés insolubles dans l'eau choisis parmi :
   - V₂O₅, CeO₂, Nb₂O₅
   - WO₃, Na₄W₁₀O₃₂
   - MoO₃, MoS₂, RuO₂, Mn₂O₃, Bi₂O₃.
   - Fe₂O₃, Co₃O₄
   - CuInS₂, CuIn₅S₈,
   - les métaux tels que Ag, Cu, Au, Ni
2) les sulfures, les carbures et les phosphures insolubles dans l'eau choisis parmi:
   GaP, SiC, CdS et TiS
3) les composites insolubles dans l'eau choisis parmi les composites suivants : TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂ enrobé en surface de composés comprenant au moins un éléments choisi parmi Al, Zn, Zr, Cr, V, Nv, Fe, Cu, Co, Ni, Mn.

La présente invention a également pour objet un procédé de traitement cosmétique des cheveux humains destinées à diminuer et/ou à éliminer les composés organiques indésirables sur les cheveux humains , notamment le sébum, ledit procédé étant caractérisé par le fait qu'il comprend les étapes suivantes :
(i) on applique sur les cheveux, notamment secs une composition comprenant un photocatalyseur tel que défini ci-dessous dans un milieu cosmétiquement acceptable,
(ii) éventuellement on masse les cheveux à l'aide des doigts ,
(iii) éventuellement on sèche les cheveux.

La présente invention a également pour objet un procédé de traitement des cheveux pour diminuer et/ou retarder le regraissage des cheveux humains, ledit procédé étant caractérisé par le fait qu'il comprend les étapes suivantes :
(i) on applique sur les cheveux, notamment secs une composition comprenant un photocatalyseur tel que défini ci-dessous dans un milieu cosmétiquement acceptable,
(ii) éventuellement on masse les cheveux à l'aide des doigts ,
(iii) éventuellement on sèche les cheveux.

Au sens de la présente invention, on entend par photocatalyseur un catalyseur dont l'activité consiste à accélérer une réaction en présence d'un rayonnement électromagnétique de longueur d'onde comprise entre 200 et 700 nanomètres.

La version du tableau de la classification périodique utilisée est la version CAS (Chemical Associated Society). Il est notamment inclus dans l'ouvrage "The Merck Index" 11ed. 1989.

Par "insoluble dans l'eau", on entend tout composé qui, à une concentration supérieure ou égale à 0,1% en poids dans l'eau à 25°C, ne forment pas à l'oeil nu une solution isotrope transparente.

Conformément à l'utilisation selon l'invention, les photocatalyseurs sont choisis parmi :
1) les composés insolubles dans l'eau comprenant au moins un élément choisis parmi le vanadium, le niobium, le tantale, le molibdène, le tungstène, le manganèse, le fer, le ruthénium, le cobalt, le nickel, le cuivre, l'argent, l'or, le bismuth, le titane, le zinc, le zirconium.
2) les sulfures, les carbures et les phosphures insolubles dans l'eau comprenant au moins un élément des colonnes suivantes de la classification périodique des éléments : IIb, IIIa, IVa, IVb
3) les composites insolubles dans l'eau comprenant au moins un élément choisi parmi :
   - Titane, Zinc, Silicium, Aluminium, Magnésium, Sodium, Calcium, Zirconium.

Parmi les composés de la famille 1), on peut notamment citer les composés suivants :
- V₂O₅, CeO₂, Nb₂O₅
- WO₃, Na₄W₁₀O₃₂
- MoO₃, MoS₂, RuO₂, Mn₂O₃, Bi₂O₃,
- Fe₂O₃, Co₃O₄
- CuInS₂, CuIn₅S₈,
- les métaux tels que Ag, Cu, Au, Ni

Parmi les composés de la famille 2), on peut notamment citer GaP, SiC, CdS, TiS.

Par composite, on entend au sens de la présente invention, une combinaison macroscopique de deux ou plusieurs matériaux. Le caractère macroscopique exclut les alliages.

Parmi les composés de la famille 3), on peut notamment citer les composites suivants :
TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂ enrobé en surface de composés comprenant au moins un éléments choisi parmi Al, Zn, Zr, Cr, V, Nv, Fe, Cu, Co, Ni, Mn.

Les photocatalyseurs utilisables dans les compositions selon l'invention de la famille 1), sont choisis parmi :
- V₂O₅, Nb₂O₅
- RuO₂, Mn₂O₃, Bi₂O₃,
- Co₃O₄
- CuInS₂, CuIn₅S₈.

Les photocatalyseurs utilisables dans les compositions selon l'invention de la famille 2), sont choisis parmi :
GaP, CdS et TiS.

Parmi les composés de la famille 3) utilisables dans les compositions de l'invention on peut notamment citer les composites suivants :
TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂ enrobé en surface de composés comprenant au moins un éléments choisi parmi V, Nv, Cu, Mn.

Le photocatalyseur est généralement présent dans la composition en une quantité comprise entre 0,1 et 20% en poids par rapport au poids total de la composition et de préférence entre 0,2 et 10% en poids et plus particulièrement de 0,2 à 3% en poids.

Le milieu cosmétiquement acceptable peut être constitué uniquement par de l'eau ou par un mélange d'eau et d'un ou plusieurs solvants cosmétiquement acceptables ou par un ou plusieurs solvants cosmétiquement acceptables, tel qu'un alcool inférieur en C₁-C₄, comme l'éthanol, l'isopropanol, le tertiobutanol, le n-butanol; les alkylèneglycols comme le propylèneglycol, les éthers de glycols. L'eau représente de préférence de 30 à 98% en poids et de préférence de 50 à 98% en poids par rapport au poids total de la composition.

Le pH des compositions utilisées selon l'invention est généralement compris entre 2 et 12.

La composition de l'invention peut également contenir au moins un additif choisi parmi les épaississants, les parfums, les agents nacrants, les conservateurs, les filtres solaires, les tensioactifs anioniques, non ioniques, amphotères ou cationiques, les polymères anioniques ou non ioniques ou amphotères, les polymères cationiques, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, les provitamines telles que le panthénol, les silicones, les huiles végétales, les huiles minérales et les huiles de synthèse, les agents antipelliculaires et tout autre additif classiquement utilisé dans le domaine cosmétique qui n'affecte pas la stabilité et les propriétés des compositions selon l'invention.

Ces additifs éventuellement sont présents dans la composition selon l'invention dans des proportions pouvant aller de 0,001 à 50% en poids par rapport au poids total de la composition. La quantité précise de chaque additif est déterminée facilement par l'homme du métier selon sa nature et sa fonction.

L'application de la composition peut se faire avec les mains, avec un pulvérisateur, avec un aérosol, avec un embout applicateur, avec un peigne dispensateur ou avec une serviette imprégnée de la composition. Cette application peut être ou non suivie d'un rinçage.

Dans les exemples qui suivent, donnés à titre illustratif et non limitatif, on donne une composition concrète conforme à l'invention.

### EXEMPLE :

On a utilisé une composition contenant 5% en poids de nanoparticules d'Argent (15 nm) en solution aqueuse colloidale, commercialisée par la société ADVANCED NANOPRODUCTS sous la référence "Ag Sol"

On a déposé cette solution à raison de 1 g sur une mèche propre de cheveux naturels de 2.7 g, soit une quantité de 18.5 mg d'argent par g de cheveux.

La mèche est séchée au casque pendant 30 minutes à 60°C.

Une mèche placebo est également réalisée en utilisant de l'eau.

Les mèches sont exposée pendant 24 heures à la lumière du jour.

De fines goutelettes de sébum artificiel sont ensuite vaporisées sur la mèche (TO).

La mèche est ensuite exposée pendant 6 heures (T6), au SUN TEST (Hanau) équipée d'une lampe XENON permettant le passage des rayons UV dans une gamme de longueur d'ondes comprises entre 300 et 830 nm. La lampe a été refroidie pendant le test au moyen d'un flux d'air d'environ 60m³/heure. Un second flux d'air d'environ 60m³/heure refroidissait les mèches. L'intensité de la lumière au niveu des mèches est d'environ 150 lux. L'intenité d'irradiation est d'environ 830 W/m² 5%RH)). Une mèche témoin est laissée dans une boîte à gants obscure à l'abri de toute source de rayonnement.

| | **Produit appliqué sur la mèche** | **Application de sébum** | **Exposition des mèches** |
|---|---|---|---|
| Mèche 1 | Nanoparticules d'Argent | oui | 6 heures de SUN TEST |
| Mèche 2 | Nanoparticules d'Argent | oui | 6 heures OBSCURITE |
| Mèche 3 | Placebo (Eau) | oui | 6 heures de SUN TEST |
| Mèche 4 | Placebo (Eau) | oui | 6 heures OBSCURITE |
| Mèche 5 | Rien | oui | 6 heures de SUN TEST |
| Mèche 6 | Rien | oui | 6 heures OBSCURITE |

Les mèches sont examinées par les évaluateurs, après les 6 heures d'exposition, suivi de 15 minutes de conditionnement à température ambiante.

La comparaison sensorielle des mèches a été réalisée selon un test triangulaire par 10 sujets.

La mèche 1 paraît significativement moins grasse (au toucher et visuellement) et plus propre que les mèches 2 à 6.

## Revendications

1. Utilisation d'un photocatalyseur dans des compositions capillaires destinées à diminuer et/ou à éliminer les composés organiques indésirables sur les cheveux humains, ledit photocatalyseur étant choisi parmi :
1) les composés insolubles dans l'eau choisis parmi :
- V₂O₅, CeO₂, Nb₂O₅
- WO₃, Na₄W₁₀O₃₂
- MoO₃, MoS₂, RuO₂, Mn₂O₃, Bi₂O₃,
- Fe₂O₃, Co₃O₄
- CuInS₂, CuIn₅S₈,
- les métaux tels que Ag, Cu, Au, Ni
2) les sulfures, les carbures et les phosphures insolubles dans l'eau choisis parmi :
GaP, SiC, CdS et TiS
3) les composites insolubles dans l'eau choisis parmi les composites suivants : TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂ enrobé en surface de composés comprenant au moins un éléments choisi parmi Al, Zn, Zr, Cr, V, Nv, Fe, Cu, Co, Ni, Mn.

2. Utilisation selon la revendication 1, **caractérisée en ce que** le composé organique est le sébum.

3. Utilisation selon l'une quelconque des revendications 1 à 2, **caractérisée en ce que** la quantité de photocatalyseur est comprise entre 0,1 et 20% en poids par rapport au poids total de la composition.

4. Utilisation selon l'une quelconque des revendications 1 à 3, **caractérisée en ce que** lesdites compositions capillaires comprennent un milieu aqueux ou hydroalcoolique.

5. Utilisation selon l'une quelconque des revendications 1 à 4, **caractérisée en ce que** lesdites compositions capillaires comprennent en outre un additif choisi parmi les les épaississants, les parfums, les agents nacrants, les conservateurs, les filtres solaires, les tensioactifs anioniques, non ioniques, amphotères ou cationiques, les polymères anioniques ou non ioniques ou amphotères, les polymères cationiques, les protéines, les hydrolysats de protéines, les céramides, les pseudocéramides, les acides gras à chaînes linéaires ou ramifiées en C₁₆-C₄₀ tels que l'acide méthyl-18 eicosanoique, les hydroxyacides, les vitamines, les provitamines telles que le panthénol, les silicones, les huiles végétales, les huiles minérales et les huiles de synthèse, les agents antipelliculaires.

6. Utilisation d'un photocatalyseur dans des compositions capillaires destinées à diminuer et/ou retarder le regraissage des cheveux humains, ledit photocatalyseur étant choisi parmi :
1) les composés insolubles dans l'eau choisis parmi :
- V₂O₅, CeO₂, Nb₂O₅
- WO₃, Na₄W₁₀O₃₂
- MoO₃, MoS₂, RuO₂, Mn₂O₃, Bi₂O₃,
- Fe₂O₃, Co₃O₄
- CuInS₂, CuIn₅S₈,
- les métaux tels que Ag, Cu, Au, Ni
2) les sulfures, les carbures et les phosphures insolubles dans l'eau choisis parmi:
GaP, SiC, CdS et TiS
3) les composites insolubles dans l'eau choisis parmi les composites suivants : TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂ enrobé en surface de composés comprenant au moins un éléments choisi parmi Al, Zn, Zr, Cr, V, Nv, Fe, Cu, Co, Ni, Mn.

## Claims

1. Use of a photocatalyst in hair compositions intended to decrease and/or to eliminate the unwanted organic compounds on human hair, said photocatalyst being chosen from:
1) water-insoluble compounds chosen from:
- V₂O₅, CeO₂, Nb₂O₅
- WO₃, Na₄W₁₀O₃₂
- MoO₃, MoS₂, RuO₂, Mn₂O₃, Bi₂O₃,
- Fe₂O₃, Co₃O₄
- CuInS₂, CuIn₅S₈,
- metals such as Ag, Cu, Au, Ni;
2) water-insoluble sulphides, carbides and phosphides chosen from:
GaP, SiC, CdS and TiS;
3) water-insoluble composites chosen from the following composites: TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, and TiO₂ surface-coated with compounds comprising at least one element chosen from Al, Zn, Zr, Cr, V, Nv, Fe, Cu, Co, Ni and Mn.

2. Use according to Claim 1, **characterized in that** the organic compound is sebum.

3. Use according to either one of Claims 1 and 2, **characterized in that** the amount of photocatalyst is between 0.1 and 20% by weight relative to the total weight of the composition.

4. Use according to any one of Claims 1 to 3, **characterized in that** said hair compositions comprise an aqueous or aqueous-alcoholic medium.

5. Use according to any one of Claims 1 to 4, **characterized in that** said hair compositions also comprise an additive chosen from thickeners, fragrances, pearlescent agents, preserving agents, sunscreens, anionic, nonionic, amphoteric or cationic surfactants, anionic, nonionic or amphoteric polymers, cationic polymers, proteins, protein hydrolysates, ceramides, pseudoceramides, C₁₆-C₄₀ linear- or branched-chain fatty acids such as 18-methyl eicosanoic acid, hydroxy acids, vitamins, provitamins such as panthenol, silicones, plant oils, mineral oils and synthetic oils, and anti-dandruff agents.

6. Use of a photocatalyst in hair compositions intended to decrease and/or delay regreasing of human hair, said photocatalyst being chosen from:
1) water-insoluble compounds chosen from:
- V₂O₅, CeO₂, Nb₂O₅
- WO₃, Na₄W₁₀O₃₂
- MoO₃, MoS₂, RuO₂, Mn₂O₃, Bi₂O₃,
- Fe₂O₃, Co₃O₄
- CuInS₂, CuIn₅S₈,
- metals such as Ag, Cu, Au, Ni;
2) water-insoluble sulphides, carbides and phosphides chosen from:
GaP, SiC, CdS and TiS;
3) water-insoluble composites chosen from the following composites: TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, and TiO₂ surface-coated with compounds comprising at least one element chosen from Al, Zn, Zr, Cr, V, Nv, Fe, Cu, Co, Ni and Mn.

## Patentansprüche

1. Verwendung eines Photokatalysators in Zusammensetzungen zur Haarbehandlung, die zur Verringerung und/oder Beseitigung von unerwünschten organischen Verbindungen auf den menschlichen Haaren vorgesehen sind,
wobei der Photokatalysator ausgewählt ist unter
1) wasserunlöslichen Verbindungen, die ausgewählt sind unter
- V₂O₅, CeO₂, Nb₂O₅,
- WO₃, Na₄W₁₀O₃₂,
- MoO₃, MoS₂, RuO₂, Mn₂O₃, Bi₂O₃,
- Fe₂O₃, Co₃O₄,
- CuInS₂, CuIn₅S₈,
- Metallen wie Ag, Cu, Au, Ni;
2) wasserunlöslichen Sulfiden, Carbiden und Phosphiden, die ausgewählt sind unter
GaP, SiC, CdS und TiS;
3) wasserunlöslichen Kombinationen, die ausgewählt sind unter folgenden Kombinationen: TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂, das auf der Oberfläche mit Verbindungen ummantelt ist, die mindestens ein Element aufweisen, das unter Al, Zn, Zr, Cr, V, Nv, Fe, Cu, Co, Ni, Mn ausgewählt ist.

2. Verwendung nach Anspruch 1, **dadurch gekennzeichnet, dass** die organische Verbindung das Sebum ist.

3. Verwendung nach Anspruch 1 oder 2, **dadurch gekennzeichnet, dass** die Menge an Photokatalysator im Bereich von 0,1 bis 20 Gew.-% liegt, bezogen auf das Gesamtgewicht der Zusammensetzung.

4. Verwendung nach einem der Ansprüche 1 bis 3, **dadurch gekennzeichnet, dass** die Zusammensetzungen zur Haarbehandlung ein wässeriges oder wässerigalkoholisches Medium aufweisen.

5. Verwendung nach einem der Ansprüche 1 bis 4, **dadurch gekennzeichnet, dass** die Zusammensetzungen zur Haarbehandlung ferner ein Additiv enthalten, das ausgewählt ist unter Verdickungsmitteln, Parfums, Perlglanzmitteln, Konservierungsmitteln, Sonnenfiltern, anionischen, nichtionischen, amphoteren oder kationischen Tensiden, anionischen oder nichtionischen oder amphoteren Polymeren, kationischen Polymeren, Proteinen, Proteinhydrolysaten, Ceramiden, Pseudoceramiden, geradkettigen oder verzweigten C₁₆-C₄₀-Fettsäuren, wie 18-Methyleicosansäure, Hydroxysäuren, Vitaminen, Provitaminen, wie Panthenol, Siliconen, Pflanzenölen, Mineralölen und synthetischen Ölen und Mitteln gegen Schuppen.

6. Verwendung eines Photokatalysators in Zusammensetzungen zur Haarbehandlung, die zur Verringerung und/oder Verzögerung der Wiederfettung der menschlichen Haare vorgesehen sind, wobei der Photokatalysator ausgewählt ist unter
1) wasserunlöslichen Verbindungen, die ausgewählt sind unter
- V₂O₅, CeO₂, Nb₂O₅,
- WO₃, Na₄W₁₀O₃₂,
- MoO₃, MoS₂, RuO₂, Mn₂O₃, Bi₂O₃,
- Fe₂O₃, Co₃O₄,
- CuInS₂, CuIn₅S₈,
- Metallen wie Ag, Cu, Au, Ni;
2) wasserunlöslichen Sulfiden, Carbiden und Phosphiden, die ausgewählt sind unter
GaP, SiC, CdS und TiS;
3) wasserunlöslichen Kombinationen, die ausgewählt sind unter folgenden Kombinationen: TiO₂ + ZnO, TiO₂ + CuO, TiO₂ + RuO₂, TiO₂ + SnO₂, TiO₂ + MoO₃, TiO₂ + WO₃, TiO₂ + GeO₂, TiO₂ + MoO₃, TiO₂ + WO₃, TiO₂ + GeO₂, TiO₂, das auf der Oberfläche mit Verbindungen ummantelt ist, die mindestens ein Element aufweisen, das unter Al, Zn, Zr, Cr, V, Nv, Fe, Cu, Co, Ni, Mn ausgewählt ist.
